(19) Europäisches Patentamt
European Patent Office
Office européen des brevets

(11) Publication number: **0 062 717**
**B1**

(12) **EUROPEAN PATENT SPECIFICATION**

(45) Date of publication of patent specification: **03.07.85**

(51) Int. Cl.$^4$: **C 07 C 120/06,** C 07 C 121/75

(21) Application number: **81305154.7**

(22) Date of filing: **30.10.81**

(54) **A process for the manufacture of aromatic cyanides.**

(30) Priority: **09.04.81 GB 8111086**

(43) Date of publication of application:
**20.10.82 Bulletin 82/42**

(45) Publication of the grant of the patent:
**03.07.85 Bulletin 85/27**

(84) Designated Contracting States:
**AT BE CH DE FR IT LI LU NL SE**

(56) References cited:
**EP-A-0 038 656**
**GB-A-1 476 073**

**JOURNAL OF ORGANIC CHEMISTRY Vol. 41, No. 14, 9 July 1976 SCHWARTZ et al. "A Convient Synthesis of o- and p-Hydroxy Substitued Phenyl-acetonitriles and Phenethylamines" pages 2502 to 2503**

(73) Proprietor: **A.H. Marks & Co. Limited**
**Wyke**
**Bradford West Yorkshire BD12 9EJ (GB)**

(72) Inventor: **Stanley, Robert Holroyd**
**52a Old Elvet**
**Durham, DH1 3HN (GB)**
Inventor: **Hindley, Nathan Chadwick**
**Correio Praia da Luz Caixa Postal 110**
**8600 Lagos (PT)**

(74) Representative: **Sanderson, Laurence Andrew et al**
**Sanderson & Co. 97 High Street**
**Colchester Essex C01 1TH (GB)**

**EP 0 062 717 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with the manufacture of aromatic cyanides. More specifically it relates to a process for the preparation of *ortho*- and *para*-hydroxybenzyl cyanides, which may optionally be further substituted in the aromatic nucleus, from the corresponding *ortho*- and *para*-hydroxybenzyl alcohols.

It is well known that *ortho*- and *para*-hydroxybenzyl alcohols, due to the activating effect of the phenolic hydroxyl group, cannot be converted into the corresponding benzyl cyanides (or at least not in any practical yield) by the conventional techniques of halogenation followed by reaction with sodium cyanide in a suitable solvent. In the particular case of *ortho*- and *para*-hydroxybenzyl alcohol it is however possible to achieve the desired conversion under reaction conditions which do not involve the use of acidic reagents. For example para-hydroxybenzyl alcohol may be converted into the corresponding cyanide in 67% yield using a 4% solution of the starting material in dimethyl formamide and heating with sodium cyanide for 20 hours within a temperature range of 110—130° C (Schwartz et. al., J. Org. Chem. 1976 *41* 2502—3). Also para-hydroxybenzyl alcohol may first be converted to the corresponding diacetate with acetic anhydride and the diacetate then reacted with potassium cyanide in methanolic solution to give a 70% yield of product (Hayashi et. al., Bull. Inst. Chem. Res. Kyoto Univ. 1974 *52* 514). These reactions have the disadvantage that in the former a large excess of an expensive solvent is used, whilst in the latter the corresponding diacetate must be prepared using an expensive reagent.

British patent specification 1476073 discloses that certain *ortho*- and *para*-hydroxybenzyl cyanides are obtained when the corresponding hydroxybenzyl alcohols are reacted with hydrogen cyanide in water or an inert organic solvent at temperatures between 80 and 190° C. Solvents which can be used are stated to be water and all inert organic solvents, especially polar aprotic solvents, such as N,N-dimethylformamide, dimethylsulphoxide, tetrahydrothiophene-S-dioxide, N-methylpyrrolidone, tetramethylurea, hexamethylphosphoric acid triamide and methyl cyanide. Dimethylsulphoxide is stated to be preferred.

Clearly it would be desirable to provide a synthesis which was generally more convenient than the known processes, for example in that it could proceed under mild conditions, could employ readily obtainable reagents and solvents which are relatively less expensive, could give good yields, could employ simplified isolation processes, need not isolate intermediates or the like. A process has now been discovered which can be adapted to offer such advantages.

Accordingly, the present invention provides a process for the preparation of an *ortho*- or *para*-hydroxybenzyl cyanide which process comprises the reaction in methanol and/or ethanol as solvent of an *ortho*- or *para*-hydroxybenzyl alcohol with a cyanide and an ester capable of esterifying the benzyl hydroxyl group.

Esters for use in this invention will normally be primary alcohol aliphatic esters. Apt esters for use in the process of the present invention include acetate and formate esters.

Surprisingly it has been discovered that the use of formate esters offer considerable advantages over the use of acetate esters, for example higher yields may be obtained when using formate esters. Particularly favoured esters for use include methyl formate and ethyl formate.

Suitable cyanides for use in the process of the present invention include the cyanides of the alkali metals. The basic nature of these cyanides is believed to aid the reaction. Preferred cyanides for use in this invention are sodium or potassium cyanide.

Generally the reaction is carried out at an elevated temperature and is most suitably performed under reflux.

The process may be carried out by mixing together the reactants in the solvent and stirring whilst maintaining the reactants at an elevated temperature. Preferably the temperature is that at which the mixture refluxes.

Under the reaction conditions hydroxyl groups of the *ortho*- and *para*-hydroxybenzyl alcohol may be successively esterified by ester transfer involving base catalysed alcoholysis of the ester in the reaction mixture by the solvent. The base catalyst may be the alkali metal cyanides. The following reaction scheme illustrates the process with respect to *para*-hydroxybenzyl alcohol in the presence of a cyanide such as sodium and a formate such as methyl formate in a solvent such as methanol.

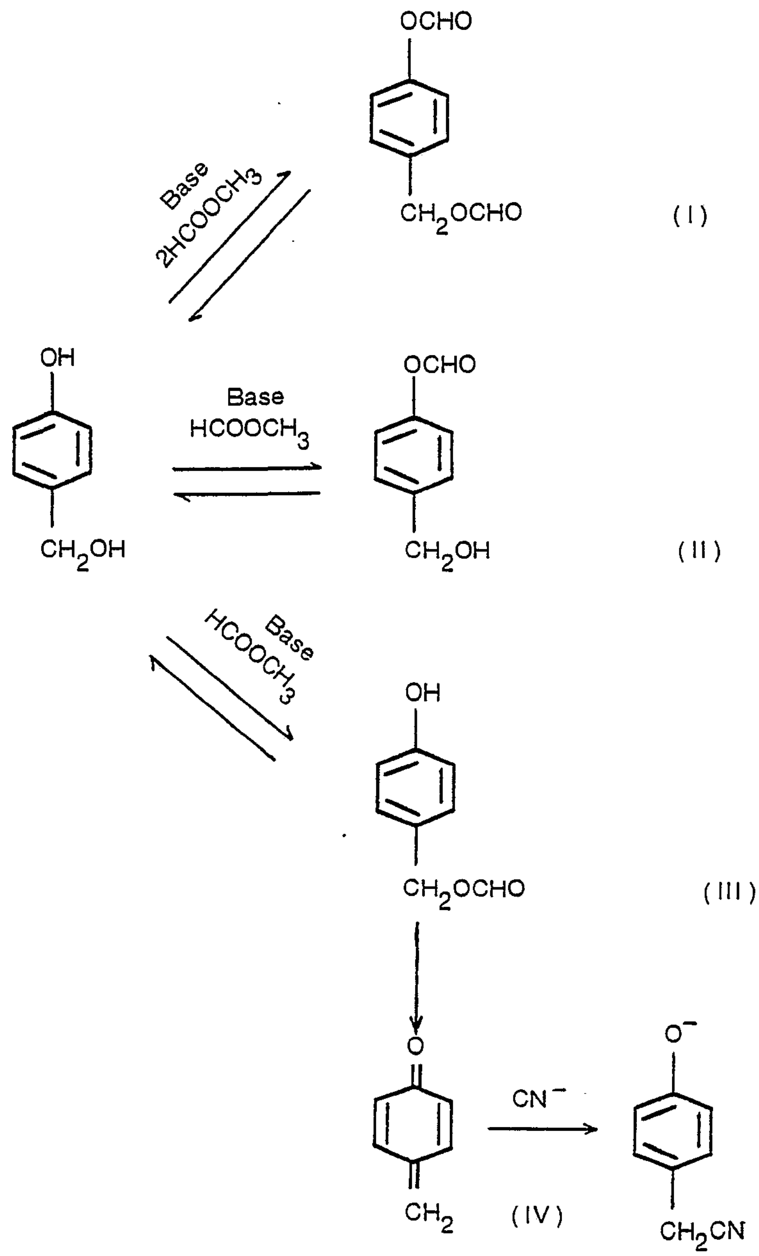


(I)

(II)

(III)

(IV)

(V)

A mixture of the diester (I) and the monoesters (II) and (III) is formed. Only in the case of the monoester (III) with a free phenol function is it possible to generate the reactive quinone methide (IV) which by base catalysis can add cyanide and form the desired product. The reaction system has the advantage that the unreactive esters (I) and (II) may be converted to the reactive ester (III) due to the equilibrium nature of the system. The ester (III) is removed from the system by the irreversible reaction to give compounds (IV) and (V), the unreactive esters may be gradually converted to the reactive ester and may thereby give advantageous yields of the desired product. The use of the formate ester in the reaction is particularly advantageous.

From the aforementioned it is clear that in a further aspect this invention also provides a process for the preparation of *ortho-* or *para*-hydroxybenzyl cyanide which comprises the reaction in methanol and/or ethanol as solvent of an *ortho-* or *para*-hydroxybenzyl formate with sodium or potassium cyanide.

Generally this form of the process of the present invention is performed at elevated temperature and is most suitably performed under reflux. Whereas the formate employed in this aspect of the invention may be preformed if desired, most suitably it is formed *in situ* by transesterification of the hydroxybenzyl alcohol with a formate ester such as methyl or ethyl formate.

The process of this invention is normally carried out in a medium which is a mixture of the primary alcohol (methanol and/or ethanol) and an aliphatic ester. The proportion of primary alcohol to aliphatic ester in the reaction medium is to some extent dictated by the velocity of the reaction. At low or high proportions of primary alcohol to aliphatic ester the reaction velocity may be too slow resulting in the

production of unwanted by-products. Suitably the ratio of primary alcohol to ester is from 1:1 to 4:1 by volume. A preferred mixture would involve 2 to 4 parts alcohol to 1 part ester volume.

It is preferred that the ester used in the reaction medium is a formate ester.

Clearly it is a considerable advantage that the process of this invention may employ relatively inexpensive materials such as methanol, ethanol, methyl formate and ethyl formate. The use of sodium or potassium cyanides in the process of this invention is also desirable in view of their availability and relatively inexpensive costs.

On completion of a reaction of this invention the excess solvent and reagent and recoverable from the reaction mixture. Suitably this recovery may be carried out using distillation. The reaction products and any unchanged starting material may be separated from inorganic salts by acidification with dilute mineral acid for example sulphuric acid and extraction of the organic materials by partition into an organic solvent. Suitable organic solvents include methylene chloride and chloroform. the combined organic extracts may be treated as follows. Firstly the organic solvent is removed by evaporation and the pure product of the process of the present invention isolated by distillation under reduced pressure.

As will be appreciated by the skilled chemist, the process of this invention may also be carried out on ortho- or para-hydroxybenzyl alcohols which are substituted on the aromatic nucleus (provided of course that the substitution is not one which prevents reaction). Suitable substituents include lower alkoxy groups such as the methoxy group. A hydroxylbenzyl alcohol of this type is vanillyl alcohol.

The following Examples illustrate the invention.

Example 1

*Preparation of para-hydroxybenzyl cyanide*

A mixture of 4.96 g para-hydroxybenzyl alcohol 2.8 g sodium cyanide 18 ml. methanol and 6 ml. of methyl formate is heated with stirring under reflux for a period of 70 minutes. The reaction mixture is distilled under partial vacuum to recover starting material, and the residue stirred with 20 ml. water and sulphuric acid added until the mixture is no longer alkaline. The product is now isolated by extracting 3 times each time with 15 ml. methylene chloride. The combined solvent extracts are washed with 10 ml. aqueous sodium bicarbonate and the solvent is evaporated finally under vacuum at a temperature of 60° C. There is obtained 5.11 g of a crude para-hydroxybenzyl cyanide corresponding to a weight yield of 96% of theory. On simple distillation under oil pump vacuum there is obtained a pale yellow fraction m.pt 65° C corresponding to a weight yield equivalent to 93% of theory. Melting point data indicates that this material has an absolute purity of 97%.

Example 2

*Preparation of para-hydroxybenzyl cyanide*

A mixture of 4.96 g. para-hydroxybenzyl alcohol 2.4 g sodium cyanide 12 ml. ethanol and 12 ml. ethyl formate is heated with stirring under reflux for a period of ninety minutes. The reaction product is then subjected to the same isolation process as in Example 1 to give a 94% yield of crude product showing a melting point of 58—61°C uncorrected.

Example 3

*Preparation of para-hydroxybenzyl cyanide*

A reaction was carried out in an analogous manner to Example 1 except that methyl acetate was used in place of methyl formate. Para-hydroxybenzyl cyanide was obtained in a less advantageous yield than in Example 1.

Example 4

*Preparation of ortho-hydroxybenzyl cyanide*

A reaction was carried out in an analogous manner to Example 1 except that ortho-hydroxybenzyl alcohol was used in place of para-hydroxybenzyl alcohol. Ortho-hydroxybenzyl cyanide was obtained in yield of 92.7% (Crude product)

Example 5

*Preparation of 4-hydroxy-3-methoxy benzyl cyanide*

A reaction was carried out in an analogous manner to Example 1 except that 4-hydroxy-3-methoxy benzyl alcohol was used in place of para-hydroxy-benzyl alcohol. 4-Hydroxy-3-methoxybenzyl cyanide was obtained in a yield of 77.4% (Crude product).

**Claims**

1. A process for the manufacture of aromatic cyanides, in which an aromatic alcohol being an *ortho-* or *para-* hydroxybenzyl alcohol, optionally further substituted in the aromatic nucleus, is reacted in methanol and/or ethanol as solvent with a cyanide reagent and an ester capable of esterifying the said aromatic alcohol, and the corresponding *ortho-* or *para-*hydroxybenzyl cyanide thus produced is recovered.

2. A process as claimed in claim 1, in which the cyanide reagent employed is sodium and/or potassium cyanide.

3. A process as claimed in claim 1 or claim 2, in which the ester is a primary alcohol aliphatic ester, preferably a formate and above all either methyl formate or ethyl formate.

4. A process as claimed in any one of the preceding claims, in which the reaction is carried out at an elevated temperature.

5. A process as claimed in any one of claims 1 to 3, in which the reaction is carried out under reflux of the solvent employed.

6. In a process for the manufacture of aromatic cyanides, the step of reacting an *ortho-* or *para-*hydroxybenzyl formate, optionally further substituted in the aromatic nucleus, in methanol and/or ethanol as solvent, with sodium and/or potassium cyanide(s), to yield the corresponding *ortho-* or *para-*hydroxybenzyl cyanide.

7. A process as claimed in claim 6, in which the reaction is carried out under reflux.

8. A process as claimed in any one of the preceding claims, in which the ratio of methanol and/or ethanol to ester is in the range of from about 1:1 to about 4:1 by volume.

9. A process for the manufacture of *ortho-* and *para-*hydroxybenzyl cyanides, optionally further substituted in the aromatic nucleus, in which:

(a) a cyanide reagent comprising sodium and/or potassium cyanide(s)

is reacted with

(b) an aromatic starting material comprising:

either (i) a mixture of an *ortho-* or *para-* hydroxybenzyl alcohol with a formate ester,

or (ii) an *ortho-* or para-hydroxybenzyl formate

in a primary alcoholic solvent comprising methanol and/or ethanol, at about reflux temperature utilizing a ratio of the primary alcohol to ester of in the range of from about 2:1 to about 4:1 by volume, so as thus to yield the desired corresponding aromatic cyanide.

10. A process as claimed in claim 9, in which after the reaction excess solvent and reactants are removed and recovered by distillation, the product thus obtained after removal of excess solvent and reactants is then separated from inorganic salts by means of extraction from an acidic aqueous medium into an organic solvent, the latter is then evaporated off and finally the resultant product is distilled under reduced pressure to yield the desired aromatic cyanide in substantially pure form.

**Patentansprüche**

1. Verfahren zur Herstellung von aromatischen Cyaniden, bei dem ein aromatischer Alkohol, bei dem es sich um einen ortho- oder para-Hydroxybenzylalkohol handelt, der gegebenenfalls zusätzlich am aromatischen Kern substituiert sein kann, in Methanol und/oder Ethanol als Lösungsmittel mit einem Cyanidreagenz und einem Ester zur Reaktion gebracht wird, der in der Lage ist, den aromatischen Alkohol zu verestern, und das so hergestellte entsprechende orth- oder para-Hydroxybenzylcyanid gewonnen wird.

2. Verfahren nach Anspruch 1, bei dem das verwendete Cyanidreagenz Natrium- und/oder Kaliumcyanid ist.

3. Verfahren nach Anspruch 1 oder 2, bei dem der Ester ein aliphatische Ester eines primären Alkohols, vorzugsweise ein Formiat und insbesondere Methylformiat oder Ethylformiat ist.

4. Verfahren nach einem der vorangehenden Ansprüche, bei dem die Reaktion bei erhöhter Temperatur durchgeführt wird.

5. Verfahren nach einem der Ansprüche 1 bis 3, bei dem die Reaktion unter Rückfluß des verwendeten Lösungsmittels durchgeführt wird.

6. Verfahren zur Herstellung von aromatischen Cyaniden gekennzeichnet durch die Stufe des Umsetzens eines ortho-oder para-Hydroxybenzylformiats, das gegebenenfalls zusätzlich am aromatischen Kern substituiert ist, in Methanol und/oder Ethanol als Lösungsmittel mit Natrium-und/oder Kaliumcyanid(en) unter Erhalt des entsprechenden ortho- oder para-Hydroxybenzylcyanids.

7. Verfahren nach Anspruch 6, bei dem die Reaktion unter Rückfluß durchgeführt wird.

8. Verfahren nach einem der vorangehenden Ansprüche, bei dem das Verhältnis von Methanol und/oder Ethanol zu Ester, bezogen auf das Volumen, im Bereich von 1:1 bis etwa 4:1 liegt.

9. Verfahren zur Herstellung von ortho- und para-Hydroxybenzylcyaniden, die gegebenenfalls zusätzlich am aromatischen Kern substituiert sind, bei dem:

(a) ein Cyanidreagenz, das Natrium- und/oder Kalium-cyanid(e) umfaßt,

zur Reaktion gebracht wird mit:

(b) einem aromatischen Ausgangsmaterial, das enthält: entweder (i) eine Mischung eines ortho- oder para-Hydroxybenzylalkohols mit einem Formiatester,

oder (ii) ein ortho- oder para-Hydroxybenzylformiat in einem primären Alkohollösungsmittel, das Methanol und/oder Ethanol umfaßt, bei etwa Rückflußtemperatur unter Verwendung eines Verhälttnisses von primärem Alkohol zu Ester, bezogen auf das Volumen, im Bereich von etwa 2:1 bis etwa 4:1, so daß sich das gewünschte entsprechende aromatische Cyanid ergibt.

10. Verfahren nach Anspruch 9, bei dem nach der Reaktion überschüssiges Lösungsmittel und überschüssige Reaktanden durch Destillation entfernt und zurückgewonnen werden, das so nach der Entfernung von überschüssigem Lösungsmittel und überschüssigen Reaktanden erhaltene Produkt dann von den anorganischen Salzen durch Extraktion von einem sauren wässrigen Medium in ein organisches

Lösungsmittel getrennt wird, letzteres dann verdamft wird und schließlich das resultierende Produkt unter verringertem Druck destilliert wird, um das gewünschte aromatische Cyanid in im wesentlichen reiner Form zu ergeben.

**Revendications**

1. Procédé de préparation des cyanures aromatiques, dans lequel on fait réagir un alcool aromatique, soit un alcool *ortho-* ou *para-* hydroxybenzylique, eventuellement substitué encore dans le noyau aromatique, au sein de methanol et/ou éthanol à titre de solvant, avec un réactif de cyanure et un ester capable d'esterifier le dit alcool aromatique, et on recupera le cyanure *ortho-* ou *para-* hydroxybenzylique correspondant ainsi produit.

2. Procédé selon la revendication 1 dans lequel le réactif de cyanure que l'on utilise est le cyanure de sodium et/ou de potassium.

3. Procédé selon la revendication 1 ou 2, dans lequel l'ester est un ester aliphatique d'un alcool primaire, de préférence un formiate, et surtout, soit formiate methylique ou formiate éthylique.

4. Procédé selon une quelconque des revendications précédentes, dans lequel on met la réaction en oeuvre à une température élevée.

5. Procédé selon une quelconque des revendications 1 à 3, dans lequel on met la réaction en oeuvre au reflux du solvant utilisé.

6. Dans un procédé de préparation des cyanures aromatiques, le stade de faire réagir un formiate *ortho-* ou *para-* hydroxybenzylique, eventuellement substitué encore dans le noyau aromatique, au sein de methanol et/ou éthanol à titre de solvant, avec un cyanure de sodium et/ou de potassium, pour obtenir le cyanure *ortho-* ou *para-* hydroxybenzylique.

7. Procédé selon la revendication 6, dans lequel on met la réaction en oeuvre au reflux.

8. Procédé selon une quelconque des revendications précédentes, dans lequel le rapport du methanol et/ou de l'éthanol à l'ester est inclus entre environ 1:1 jusqu'à environ 4:1 par volume.

9. Procédé de préparation des cyanures *ortho-* ou *para-* hydroxybenzyliques, eventuellement substitués encore dans le noyau aromatique, dans lequel on fait réagir.

(a) un réactif de cyanure comprenant le cyanure (s) de sodium et/ou de potassium

avec:

(b) une matière aromatique de départ comprenant:

soit (i) un mélange d'un alcool *ortho-* ou *para*-hydroxybenzylique avec un ester de formiate

ou (ii) un formiate *ortho-* ou *para-* hydroxybenzylique dans un solvant d'un alcool primaire comprenant methanol et/ou éthanol, à environ la température de reflux en utilisant un rapport de l'alcool primaire à l'ester d'environ 2:1 jusqu'à environ 4:1 per volume, pour obtenir le cyanure aromatique correspondant désiré.

10. Procédé selon la revendication 9, dans lequel, la réaction terminée, on enlève et recupère le solvant et les réactifs en excès par distillation, et qu'on sépare des sels inorganiques le produit ainsi obtenu, après l'enlèvement et la recuperation du solvant et des réactifs en excès à l'aide d'une extraction d'un milieu aqueux acidique, par un solvant organique, puis on fait évaporer ce dernier et enfin on distille le produit résultant sous pression réduit pour obtenir le cyanure aromatique désiré substantiellement pure.